# EUROPEAN PATENT APPLICATION

(11) **EP 1 621 203 A1**
(43) Date of publication of application: **01.02.2006**
(21) Application number: 04291909.2
(22) Date of filing: 27.07.2004
(51) Int. Cl.: A61K 38/04, A61K 38/16, A61K 39/085, C07K 14/31

(54) **RNAIII-inhibiting peptides for treating HIV infection**

(71) Applicant: Genoscience, 13006 Marseille (FR)
(72) Inventor: Mouhat, Stéphanie, 77500 Chelles (FR); Sabatier, Jean-Marc, 13790 Rousset (FR); Halfon, Philippe, 13018 Marseille (FR)
(74) Representative: Tezier Herman, Béatrice

(57) **Abstract**

The present invention relates to peptides or derivatives thereof, pharmaceutical compositions comprising the same and uses thereof to treat a viral infection, in particular retroviral (e.g. HIV) infection. The invention specifically discloses novel chemical compounds as active ingredients to treat such infection, in particular HIV infection.

SEQUENCE LISTING

<110>  GENOSCIENCE

<120>  Compounds and methods for treating Human Immunodeficiency Virus
infection.

<130>  B0291EP

<160>  11

<170>  PatentIn version 3.1

<210>  1
<211>  7
<212>  PRT
<213>  Artificial sequence

<220>
<223>  antiviral polypeptide
<220>
<221>  MISC_FEATURE
<222>  (2)..(2)
<223>  Xaa = any amino acid

<220>
<221>  MISC_FEATURE
<222>  (4)..(4)
<223>  Xaa = any amino acid

<400>  1
Tyr Xaa Pro Xaa Thr Asn Phe
1               5

<210>  2

<211>  7
<212>  PRT
<213>  artificial sequence

<220>
<223>  antiviral polypeptide
<400>  2
Tyr Ser Pro Ile Thr Asn Phe
1               5

<210>  3
<211>  7
<212>  PRT
<213>  artificial sequence

<220>
<223>  antiviral polypeptide
<400>  3
Tyr Ser Pro Cys Thr Asn Phe
1               5

<210>  4
<211>  7
<212>  PRT
<213>  artificial sequence

<220>
<223>  antiviral polypeptide
<400>  4
Tyr Ser Pro Trp Thr Asn Phe
1               5

<210>  5
<211>  7
<212>  PRT
<213>  artificial sequence

<220>

<223>  antiviral polypeptide

<400>  5

Tyr Lys Pro Ile Thr Asn Phe
1               5

<210>  6

<211>  21

<212>  DNA

<213>  artificial sequence


<220>

<223>  isolated DNA molecule

<400>  6
tattcgccgt ggaccaattt t                                                21

<210>  7

<211>  7

<212>  PRT

<213>  artificial sequence


<220>

<223>  antiviral polypeptide

<220>

<221>  MOD_RES

<222>  (7)..(7)

<223>  amidation


<400>  7

Tyr Ser Pro Ile Thr Asn Phe
1               5

<210>  8

<211>  7

<212>  PRT

<213>  artificial sequence

<220>

<223>  antiviral polypeptide

<220>

<221>  MOD_RES

<222>  (7)..(7)

<223>  amidation

<400>  8

Tyr Ser Pro Cys Thr Asn Phe
1               5

<210>  9

<211>  7

<212>  PRT

<213>  artificial sequence

<220>

<223>  antiviral polypeptide

<220>

<221>  MOD_RES

<222>  (7)..(7)

<223>  amidation

<400>  9

Tyr Ser Pro Trp Thr Asn Phe
1               5

<210>  10

<211>  7

<212>  PRT

<213>  artificial sequence

<220>

<223>  antiviral polypeptide

<220>

<221>  LIPID

<222>  (7)..(7)

<223>  valerate_NH2


<400>  10

Tyr Ser Pro Trp Thr Asn Phe
1               5

<210>  11

<211>  7

<212>  PRT

<213>  artificial sequence


<220>

<223>  antiviral polypeptide

<220>

<221>  MOD_RES

<222>  (7)..(7)

<223>  amidation


<400>  11

Tyr Lys Pro Ile Thr Asn Phe
1               5

## Description

### FIELD OF THE INVENTION

The present invention relates to peptides or derivatives thereof, pharmaceutical compositions comprising the same and uses thereof to treat a viral infection, in particular retroviral infection, such as Human Immunodeficiency Virus (HIV) infection. The invention specifically discloses novel chemical compounds as active ingredients to treat such infection.

### BACKGROUND OF THE INVENTION

The human immunodeficiency virus (HIV) has been implicated as the primary cause of the slowly degenerative immune system disease termed acquired immune deficiency syndrome (AIDS). There are at least two distinct types of HIV: HIV-1 and HIV-2. Further, a large amount of genetic heterogeneity exists within populations of each of these types. Infection of human CD-4 T-lymphocytes with an HIV virus leads to depletion of the cell type and eventually to opportunistic infections, neurological dysfunctions, neoplastic growth, and ultimately death. HIV is a member of the lentivirus family of retroviruses. Retroviruses are small enveloped viruses that contain a diploid, single-stranded RNA genome, and replicate via a DNA intermediate produced by a virally-encoded reverse transcriptase, an RNA-dependent DNA polymerase. Other retroviruses include, for example, HCV, HBC (hepatitis B virus), cytomegalovirus, oncogenic viruses, such as human T-cell leukemia viruses (HTLV-I,-II,-III), and feline leukemia virus.
The HIV viral particle consists of a viral core, composed of capsid proteins, that contains the viral RNA genome and those enzymes required for early replicative events. Myristylated Gag protein forms an outer viral shell around the viral core, which is, in turn, surrounded by a lipid membrane enveloped derived from the infected cell membrane. It is now well known that cells can be infected by HIV through a process by which fusion occurs between the cellular membrane and the viral membrane. The HIV envelope glycoprotein gp160 is synthesised as a precursor that is cleaved into the receptor binding gp120, and the fusogenic transmembrane gp41. After cleavage, both glycoproteins remain non-covalently associated to each other at the viral surface and are structurally and functionally coupled. The gp41 plays a critical role in entry of HIV into target cells by mediating the fusion between the viral lipid membrane and the cell plasma membrane. The cytoplasmic domain of gp41 is implicated in several HIV key functions such as viral infectivity, replication, transmission, and associated cytopathogenicity.
Although considerable effort is being put into the successful design of effective therapeutics, currently no curative anti-retroviral drugs against AIDS exist. In attempts to develop such drugs, several stages of the HIV life cycle have been considered as targets for therapeutic intervention. For example, virally encoded reverse transcriptase has been one focus of drug development. A number of reverse-transcriptase-targeted drugs, including 2',3'-dideoxynucleoside analogs, such as AZT, ddC, ddI have been developed which have been shown to be active against HIV. While beneficial, these analogs are not curative, probably due to the rapid appearance of drug resistant HIV mutants. Moreover, the drugs often exhibit toxic side effects such as bone marrow suppression, vomiting, and liver function abnormalities.
Among the diverse antiviral strategies that have been developed so far, one is based on peptides derived from functionally important domains of specific HIV proteins. This strategy has proven successful to generate some potent antiviral peptides, including the 36-residue T20/Fuzeon®, a gp41-derived peptide with anti-fusogenic properties, that is currently in phase III clinical trials.
However, there is still a need for new treatments and therapies for HIV infection and related infections. Indeed, it is generally acknowledged that there is a strong and urgent need for new and highly active anti-HIV molecules.
It is therefore an object of the present invention to provide chemical compounds useful in the treatment or prevention or amelioration of one or more symptoms of HIV infection and related infections.

### SUMMARY OF THE INVENTION

The invention provides a class of chemical compounds useful as therapeutic agents for preventing or treating HIV infection or any infection due to a virus and in particular retrovirus. It is a further object to provide compositions containing at least one of these compounds useful for the treatment or prevention or amelioration of diseases related or due to an infection by a virus, in particular retrovirus, including, for example, HCV, HBC (hepatitis B virus), cytomegalovirus, and oncogenic viruses, such as human T-cell leukemia viruses (HTLV-I,-II,-III), and feline leukemia virus, and more particularly HIV-1 or HIV-2. A particular embodiment is to provide compositions containing at least one of these compounds useful for the treatment or prevention or amelioration of one or more symptoms of virus infection, in particular HIV infection. Another object is to provide methods for production of other antiviral agents having activity against virus, in particular retrovirus, such as HIV (Human Immunodeficiency Virus), more particularly that can inhibit transmission of virus (e.g, HIV), by using said compounds. It is a further object to provide methods for preparing the compounds. It is a further object to provide the use of these compounds for the preparation of compositions useful in the treatment, the prevention, or the amelioration of diseases related or due to an infection by a virus and their use for production of other antiviral agents having activity against HIV (Human Immunodeficiency Virus). These composition are in particular useful in the treatment, the prevention, or the amelioration of one or more of the symptoms of HIV. In particular, compounds of the invention blocks HIV's ability to infect healthy immune (CD4) cells. When used with other anti-HIV agents, compounds of the invention could reduce the amount of HIV in the blood and increase the number of CD4 cells, which has been shown to slow HIV progression in patients who have developed resistance to currently available medications.
A further object of the invention is to provide methods for inhibiting transmission of virus, in particular retrovirus, such as HIV, using the compounds of the invention.
An other object herein is to provide *in vitro* model useful for investigating retrovirus (e.g., HIV) infection and particularly the process of transmission of retrovirus (e.g., HIV).

### DETAILED DESCRIPTION OF THE INVENTION

The present invention discloses polypeptides comprising at least one amino acid sequence of the general formula (I) : YX₁PX₂TNF (I) (SEQ ID No. 1), wherein X₁ and X₂, independently from each other, are any amino acid residues (or amino acid derivatives), or any other polypeptide-mimicking molecule.

The polypeptides according to the invention show high sequence identity with antibacterial RNAIII inhibiting peptides produced by S. *Aureus* bacteria, as well as the tyrosine-based endocytic signal YSPL from the HIV-1 gp41 transmembrane envelope glycoprotein.

The polypeptides, which chemical structures are detailed below, are potent inhibitors of HIV infection, being active in the nanomolar concentration range in *in vitro* assays, as shown below. These polypeptides are able to inhibit cell-to-cell transmission of HIV (inhibition of syncytium formation) as well as virus-to-cell infection (inhibition of HIV entry into target immune T-cells, i.e. human peripheral blood lymphocytes and macrophages) *in vitro.*
The molecules are neither toxic at bioactive peptide concentrations (up to 50 micromolar), nor lethal by intracerebroventricular inoculation in C57/BL6 mice (up to 200 micrograms per mouse).
In view of that properties, the polypeptides of the invention are particularly useful as active ingredients to treat a disease associated with an infection by a virus.

The term "polypeptide" refers to an isolated polymer of amino acids without regard to the length of the polymer; thus, peptides, oligopeptides, and proteins are included within the definition of polypeptide. This term also does not specify or exclude post-expression modifications of polypeptides, for example, polypeptides which include the covalent attachment of glycosyl groups, acetyl groups, phosphate groups, lipid groups and the like are expressly encompassed by the term polypeptide. Also included within the definition are polypeptides which contain one or more analogs of an amino acid (including, for example, non-naturally occurring amino acids, amino acids which only occur naturally in an unrelated biological system, modified amino acids from mammalian systems etc.), polypeptides with substituted linkages, as well as other modifications known in the art, both naturally occurring and non-naturally occurring.
The polypeptides are preferably from three (e.g. YFP) amino acids to twenty amino acids in length, more preferably from five amino acids to fifteen in length, in particular 7 amino acids, such as the specific amino acid sequence of formula (I), in length.

The polypeptides according to the invention include also variants, fragments, analogs and derivatives thereof. Said variants, fragments, analogs and derivatives of the polypeptides described hereinabove have substantially the same functions and properties as the polypeptides defined above, in particular they can be used as active ingredients to prevent or treat a viral infection caused by strains or isolates of HIV or any other pathogenic viruses, in particular retrovirus, including, for example, HCV (hepatitis C virus), HBC (hepatitis B virus), cytomegalovirus, and oncogenic viruses, such as human T-cell leukemia viruses (HTLV-I,-II,-III), and feline leukemia virus.

The variant may be 1) one in which one or more of the amino acid residues, preferably no more than two amino acid residues (preferably among Y, P, T, N and F), are substituted with a conserved or non-conserved amino acid residue and such substituted amino acid residue may or may not be one encoded by the genetic code, or 2) one in which one or more of the amino acid residues includes a substituent group, or 3) one in which one or more of the amino acid residues is substituted by a chemical compound bound to the other amino acids of the sequence by a or covalent bond(s), or 4) one in which the polypeptide according to the invention is fused, joined or linked to another compound, whereby the compound is capable of providing some (other) useful function as apparent to one skilled in the art in a method according to the present invention ; such compounds are well known in the art to include, but are not limited to, polymers, carbohydrates, phospholipids, and lipid-fatty acid conjugates; such compounds may increase the half-life of the polypeptide (for example, polyethylene glycol), or 4) one in which the additional amino acids are fused to the polypeptide according to the invention, such as a leader or secretory sequence or a sequence which is employed for purification of the polypeptide according to the invention. Such variants are deemed to be within the scope of those skilled in the art.

A polypeptide fragment is a polypeptide having a sequence that is entirely the same as part but not all of the given polypeptide sequence.

In the case of an amino acid substitution in the amino acid sequence of a polypeptide according to the invention, one or several amino acids can be replaced by "equivalent" amino acids, preferably no more than two amino acid residues (preferably among Y, P, T, N and F).
The expression "equivalent" amino acid is used herein to designate any amino acid that may be substituted for one of the amino acids having similar properties, such that one skilled in the art of peptide chemistry would expect the secondary structure and hydropathic nature of the polypeptide to be substantially unchanged. Generally, the following five groups of amino acids represent equivalent changes:(1) A, P, G, E, D, Q, N, S, T; (2) C, S, Y, T; (3) V, I, L, M, A, F; (4) K, R, H; (5) F, Y, W, H.

A specific embodiment of a polypeptide molecule of interest according to the present invention, includes, but is not limited to, a polypeptide molecule which is resistant to proteolysis, a polypeptide in which a or the -CONH- peptide bond is modified and replaced by a (CH2NH) reduced bond, a (NHCO) retro-inverso bond, a (CH2-O) methylene-oxy bond, a (CH2-S) thiomethylene bond, a (CH2CH2) carba bond, a (CO-CH2) cetomethylene bond, a (CHOH-CH2) hydroxyethylene bond), a (N-N) bound, a E-alcene bond or also a -CH=CH-bond. The invention also encompasses a polypeptide, a fragment or a variant thereof in which at least one peptide bound has been modified as described above.
Such fragments may be "free-standing", i.e. not part of or fused to other polypeptides, or they may be comprised within a single larger polypeptide of which they form a part or region.
However, several fragments may be comprised within a single larger polypeptide.

The polypeptides according to the invention are those of formula (I) wherein X₁ and X₂ are identical or different, in particular different amino acid residues.
According to a preferred embodiment, the polypeptides according to the invention are those of formula (I) wherein :
X₁ is of hydrophilic nature, such as S, K, R, D, E, and P; in particular X₁ is S or K, more particularly S; and/or
X₂ is of hydrophobic nature, such as C, I, W, V, Y, L, and F ; in particular X₂ is C, I or W.

Polypeptides according to the invention can present either carboxylic (e.g., -COOH) or carboxyl-aminated (e.g., -CONH₂) C-terminal extremity.

Examples of polypeptides according to the invention include, but are not limited to : YSPITNF (SEQ ID No. 2), YSPCTNF (SEQ ID No. 3), YSPWTNF (SEQ ID No. 4), YKPITNF (SEQ ID No. 5), YSPITNF-NH₂ (SEQ ID No. 7), reduced (monomer) or oxidized (dimer) YSPCTNF-NH₂ (SEQ ID No. 8), YSPWTNF-NH₂ (SEQ ID No. 9), and YKPITNF-NH₂ (SEQ ID No. 11).

In a particular embodiment, the polypeptides according to the invention can present a lipid moiety, covalently or not covalently attached. The lipid moiety can be any lipid presenting from C3 to C20 carbon atoms, such as valerate or myristate, at N- or C- terminal extremity. An example of such embodiment is for instance YSPWTNF-valerate-NH₂ (SEQ ID No. 10).

According to another embodiment, polypeptides of the invention can comprise at least two or more amino acid sequences of the general formula (I), said sequences are linear- or branched- attached. The number of repetitions of the sequences of formula (I) can vary from 2 to 16, preferably from 4-8. Said repeated sequences can be interrupted (or linked) by one or several, identical or different, linker(s). Said linkers can be of different natures, including amino acid derivatives, in particular bifunctional amino acid derivatives, including K, ornithine, or any other suitable linker, such as bifunctional hydrocarbonated derivatives, including diaminobutyrate and diaminopropionate, providing said linker does not change substantially the desired effect of the polypeptides according to the present invention.

In a particular embodiment, the present invention provides new branched isolated polypeptides of the following structure : (YX₁PX₂TNF)₄-K₂-K (II), wherein X₁ and X₂ are as defined above. A polypeptide of such structure is for instance : (YSPWTNF)₄-K₂-K-NH₂.

A "polypeptide-mimicking molecule" is a term used to mean a molecule which mimicks the polypeptides as defined above by presenting substantially the same effect, in particular which is able to prevent or treat a viral infection caused by strains or isolates of HIV or any other pathogenic viruses. It may include, but is not limited to, a small molecule chemical compound, a natural or synthetic peptide, a mimetic (e.g., peptidomimetic or other mimetic), a polypeptide, an aptamer, a polymer, an oligonucleotide, an antibody or fragment thereof, a polysaccharide, a carbohydrate-containing molecule, an enzyme, an agent, a macromolecule, a metabolite, a combination thereof, and the like. In a preferred embodiment, the compound is a therapeutically deliverable substance; and in a more preferred embodiment, the compound is a therapeutically deliverable substance that can inhibit transmission of virus (e.g. HIV) to a target cell.

The amino acid residues or derivatives within the motif or part of the motif in the polypeptides according to the invention can be L- and/or D-amino acids. In particular, at least one of the amino acid residues of the polypeptides of the invention is a D-amino acid. Polypeptides containing all D-amino acid residues are also included. Example provided is : Y_{D}S_{D}P_{D}W_{D}T_{D}N_{D}F_{D}-NH₂.

Peptide sequences defined herein are represented by one-letter symbols for amino acid residues as follows:
A (alanine)
R (arginine)
N (asparagine)
D (aspartic acid)
C (cysteine)
Q (glutamine)
E (glutamic acid)
G (glycine)
H (histidine)
I (isoleucine)
L (leucine)
K (lysine)
M (methionine)
F (phenylalanine)
P (proline)
S (serine)
T (threonine)
W (tryptophan)
Y (tyrosine)
V (valine)

Nucleic acids encoding the polypeptides of the invention are also included in the scope of the invention. Such nucleic acids may be DNA or RNA. In one embodiment, an isolated DNA molecule of the invention comprises the sequence TAT TCG CCG TGG ACC AAT TTT (SEQ ID No. 6). The nucleic acid molecules of the invention may be synthetic, purified, or isolated molecules. The nucleic acid molecules can be comprised in vectors such as, but not limited to, plasmids or viruses, including expression vectors. Such techniques are well known in the art.
A particular object of this invention is a vector comprising a nucleic acid encoding a polypeptide according to the present invention. The vector may be a cloning vector or, more preferably, an expression vector, i.e., a vector comprising regulatory sequences causing expression of the polypeptide of the invention from said vector in a competent host cell.

These vectors can be used to express a polypeptide according to the invention *in vitro, ex vivo* or *in vivo.*

The vectors of this invention typically comprise a coding sequence according to the present invention "operably linked" to regulatory sequences, e.g., a promoter, a polyA, etc. The term "operably linked" indicates that the coding and regulatory sequences are functionally associated so that the regulatory sequences cause expression (e.g., transcription) of the coding sequences. The vectors may further comprise one or several origins of replication and/or selectable markers. The promoter region may be homologous or heterologous with respect to the coding sequence, and provide for ubiquitous, constitutive, regulated and/or tissue specific expression, in any appropriate host cell, including for in vivo use. Examples of promoters include bacterial promoters (T7, pTAC, Trp promoter, etc.), viral promoters (LTR, TK, CMV-IE, etc.), mammalian gene promoters (albumin, PGK, etc), and the like.

The vector may be a plasmid, a virus, a cosmid, a phage, a BAC, a YAC, etc. Plasmid vectors may be prepared from commercially available vectors such as pBluescript, pUC, pBR, etc. Viral vectors may be produced from baculoviruses, retroviruses, adenoviruses, AAVs, etc., according to recombinant DNA techniques known in the art.

A further object of the present invention resides in a recombinant host cell comprising a recombinant gene or a vector as defined above. Suitable host cells include, without limitation, prokaryotic cells (such as bacteria) and eukaryotic cells (such as yeast cells, mammalian cells, insect cells, plant cells, etc.). Specific examples include E.coli, Kluyveromyces or Saccharomyces yeasts, mammalian cell lines (e.g., Vero cells, CHO cells, 3T3 cells, COS cells, etc.) as well as primary or established mammalian cell cultures (e.g., produced from fibroblasts, embryonic cells, epithelial cells, nervous cells, adipocytes, etc.).

The present invention also relates to a method for producing a recombinant host cell expressing a polypeptide according to the present invention, said method comprising (i) introducing *in vitro* or *ex vivo* into a competent host cell a recombinant nucleic acid or a vector as described above, (ii) culturing *in vitro* or *ex vivo* the recombinant host cells obtained and (iii), optionally, selecting the cells which express the polypeptide of the invention.

Such recombinant host cells can be used for the production of polypeptides, as well as for screening of active molecules, as described below.

These cells can be maintained in suitable culture media, such as DMEM, RPMI, HAM, etc., in any appropriate culture device (plate, flask, dish, tube, pouch, etc.).

The polypeptides of the invention can be synthesized by any technique commonly known in the art or may be encoded by nucleic acid as defined above, such as RNA or DNA, delivered to the host, as described above. Alternatively, they can be synthesized on a peptide synthesizer using standard solid-phase synthesis techniques and using standard FMOC/t-butyl chemistry, as described in Merrifield R.B., Science 232, 341-347, 1986. Purification of the polypeptides can also be performed from cultures of S. aureus bacteria.

The compounds of the present invention may include salts thereof. When the compounds according to the invention are in the forms of salts, they are preferably pharmaceutically acceptable salts. Such salts include pharmaceutically acceptable acid addition salts, pharmaceutically acceptable base addition salts, pharmaceutically acceptable metal salts, ammonium and alkylated ammonium salts. Acid addition salts include salts of inorganic acids as well as organic acids. Representative examples of suitable inorganic acids include hydrochloric, hydrobromic, hydroiodic, phosphoric, sulfuric, nitric acids and the like. Representative examples of suitable organic acids include formic, acetic, trichloroacetic, trifluoroacetic, propionic, benzoic, cinnamic, citric, fumaric, glycolic, lactic, maleic, malic, malonic, mandelic, oxalic, picric, pyruvic, salicylic, succinic, methanesulfonic, ethanesulfonic, tartaric, ascorbic, pamoic, bismethylene salicylic, ethanedisulfonic, gluconic, citraconic, aspartic, stearic, palmitic, EDTA, glycolic, p-aminobenzoic, glutamic, benzenesulfonic, p-toluenesulfonic acids, sulphates, nitrates, phosphates, perchlorates, borates, acetates, benzoates, hydroxynaphthoates, glycerophosphates, ketoglutarates and the like. Further examples of pharmaceutically acceptable inorganic or organic acid addition salts include the pharmaceutically acceptable salts listed in J. Pharm. Sci. 1977, 66, 2, which is incorporated herein by reference. Examples of metal salts include lithium, sodium, potassium, magnesium salts and the like. Examples of ammonium and alkylated ammonium salts include ammonium, methylammonium, dimethylammonium, trimethylammonium, ethylammonium, hydroxyethylammonium, diethylammonium, butylammonium, tetramethylammonium salts and the like. Examples of organic bases include lysine, arginine, guanidine, diethanolamine, choline and the like.

In another embodiment, this invention provides a pharmaceutical composition comprising at least one polypeptide as defined above or at least one nucleic acid encoding the same, as defined above. In particular, the pharmaceutical composition comprises a polypeptide selected in the group consisting of YSPITNF, YSPITNF-NH₂, YSPCTNF, reduced or oxidized YSPCTNF-NH₂, YSPWTNF, YSPWTNF-NH₂, YKPITNF, YKPITNF-NH₂, YSPWTNF-valerate-NH₂, (YX₁PX₂TNF)₄-K₂-K as defined above, including (YSPWTNF)₄-K₂-K-NH₂, or Y_{D}S_{D}P_{D}W_{D}T_{D}N_{D}F_{D}-NH2.

The pharmaceutical composition generally additionally comprises a pharmaceutically acceptable vehicle or carrier. Because of their viral inhibitory activity, such pharmaceutical compositions possess utility in treating viral infection, such as HIV infection, and related disorders. In particular, such pharmaceutical compositions possess utility in inhibiting cell-to-cell transmission of retrovirus, in particular HIV, as well as virus-to-cell infection.

In yet another embodiment, the present invention discloses methods for preparing pharmaceutical compositions comprising the inventive compounds as an active ingredient.

The present invention provides polypeptides that are useful as inhibitors of viral infection, in particular as inhibitors of retroviral infection, including, for example, HCV (hepatitis C virus), HBC (hepatitis B virus), cytomegalovirus, and oncogenic viruses, such as human T-cell leukemia viruses (HTLV-I,-II,-III), and feline leukemia virus, and more particularly HIV-1 or HIV-2.
These polypeptides are more particularly able to inhibit cell-to-cell transmission of virus (e.g. HIV) (in particular, inhibition of syncytium formation) as well as virus-to-cell infection (inhibition of HIV entry into target immune T-cells, i.e. human peripheral blood lymphocytes and macrophages).

Another embodiment of the invention discloses the use of the pharmaceutical compositions disclosed above for treatment of diseases, in particular for treating diseases related or due to an infection by a virus, in particular retrovirus, including, for example, HCV, HBC, cytomegalovirus, and oncogenic viruses, such as human T-cell leukemia viruses (HTLV-I,-II,-III), and feline leukemia virus, and more particularly HIV-1 or HIV-2. The method comprises administering a therapeutically effective amount of the inventive pharmaceutical composition to a mammal (in particular a patient) in need of such a treatment.

The present invention relates to a method for the treatment of a disease, and in particular a disease related or due to an infection by a virus, in particular retrovirus, including, for example, HCV, HBC, cytomegalovirus, and oncogenic viruses, such as human T-cell leukemia viruses (HTLV-I,-II,-III), and feline leukemia virus, and more particularly HIV-1 or HIV-2. More particularly, the present invention relates to a method to prevent or treat HIV infection and the like, such as the complications of HIV infection, in particular AIDS, comprising administering to a mammal (in particular a patient) in need of such treatment an effective amount of at least one polypeptide of the present invention as defined herein.

A further object of this invention is the use of an effective amount of at least one polypeptide or a nucleic acid encoding the same as defined above, for the preparation of pharmaceutical composition for the treatment of a disease associated or due to an infection by a virus, in particular retrovirus, including, for example, HCV, HBC, cytomegalovirus, and oncogenic viruses, such as human T-cell leukemia viruses (HTLV-I,-II,-III), and feline leukemia virus, and more particularly HIV-1 or HIV-2. More particularly, the pharmaceutical composition is intended to prevent or treat a disease associated with HIV infection.

Because of their anti-viral properties and particularly their HIV inhibitory transmission activity, the polypeptides of this invention are suitable for treating a variety of complications due to retroviral infection, in particular HIV.
In this regard, "treatment" or "treating" include both therapeutic and prophylactic treatments. Accordingly, the compounds may be used at very early stages of a disease, or before early onset, or after significant progression.
The polypeptides of this invention are particularly suited for the treatment of HIV and complications thereof, in particular AIDS.

In the pharmaceutical compositions, uses and methods of the present invention, the active ingredients are as described above, including the defined particular embodiments.

The active ingredients will typically be administered in admixture with suitable carrier materials suitably selected with respect to the intended form and administration, i.e. oral tablets, capsules (either solid-filled, semi-solid filled or liquid filled), powders for constitution, oral gels, elixirs, dispersible granules, syrups, suspensions, and the like, and consistent with conventional pharmaceutical practices.
The polypeptides or nucleic acids encoding the same may thus be formulated in various forms, including solid and liquid forms, such as tablets, capsules, gel, syrup, powder, aerosol, suppositories etc.
The compositions of this invention may contain physiologically acceptable diluents, fillers, lubricants, excipients, solvents, binders, stabilizers, and the like. Diluents that may be used in the compositions include but are not limited to dicalcium phosphate, calcium sulphate, lactose, cellulose, kaolin, mannitol, sodium chloride, dry starch, powdered sugar and for prolonged release tablet-hydroxy propyl methyl cellulose (HPMC). The binders that may be used in the compositions include but are not limited to starch, gelatin and fillers such as sucrose, glucose, dextrose and lactose.
Natural and synthetic gums that may be used in the compositions include but are not limited to sodium alginate, ghatti gum, carboxymethyl cellulose, methyl cellulose, polyvinyl pyrrolidone and veegum. Excipients that may be used in the compositions include but are not limited to microcrystalline cellulose, calcium sulfate, dicalcium phosphate, starch, magnesium stearate, lactose, and sucrose. Stabilizers that may be used include but are not limited to polysaccharides such as acacia, agar, alginic acid, guar gum and tragacanth, amphotsics such as gelatin and synthetic and semi-synthetic polymers such as carbomer resins, cellulose ethers and carboxymethyl chitin.
Solvents that may be used include but are not limited to Ringers solution, water, distilled water, dimethyl sulfoxide to 50% in water, propylene glycol (neat or in water), phosphate buffered saline, balanced salt solution, glycol and other conventional fluids.

Additionally, the compositions of the present invention may be formulated in a sustained release form to provide the rate controlled release of any one or more of the components or active ingredients to optimize the therapeutic effects, i.e. HIV inhibitory activity and the like. Suitable dosage forms for sustained release include layered tablets containing layers of varying disintegration rates or controlled release polymeric matrices impregnated with the active components and shaped in tablet form or capsules containing such impregnated or encapsulated porous polymeric matrices.

Liquid form preparations include solutions, suspensions and emulsions. As an example may be mentioned water or water-propylene glycol solutions for parenteral injections or addition of sweeteners and pacifiers for oral solutions, suspensions and emulsions. Liquid form preparations may also include solutions for intranasal administration.
Aerosol preparations suitable for inhalation may include solutions and solids in powder form, which may be in combination with a pharmaceutically acceptable carrier such as inert gas, e.g. nitrogen.
For preparing suppositories, a low melting wax such as a mixture of fatty acid glycerides such as cocoa butter is first melted, and the active ingredient is dispersed homogeneously therein by stirring or similar mixing. The molten homogeneous mixture is then poured into convenient sized molds, allowed to cool and thereby solidify.
Also included are solid form preparations which are intended to be converted, shortly before use, to liquid form preparations for either oral or parental administration. Such liquid forms include solutions, suspensions and emulsions.
The polypeptides and nucleic acids encoding the same of the invention may also be deliverable transdermally. The transdermal compositions may take the form of creams, lotions, aerosols and/or emulsions and can be included in the transdermal patch of the matrix or reservoir type as are conventional in the art for this purpose.

Preferably, the polypeptides and nucleic acids encoding the same are administered orally, intravenously or subcutaneously.

The dosages and dosage regimen in which the polypeptides of the invention and nucleic acids encoding the same are administered will vary according to the dosage form, mode of administration, the condition being treated and particulars of the patient being treated. Accordingly, optimal therapeutic concentrations will be best determined at the time and place through routine experimentation.

The polypeptides according to the invention and nucleic acids encoding the same can be used enterally or parenterally. Orally, the polypeptides according to the invention are suitably administered at the rate of 100 µg to 100 mg per day per kg of body weight. The required dose can be administered in one or more portions. For oral administration, suitable forms are, for example, tablets, gel, aerosols, pills, dragees, syrups, suspensions, emulsions, solutions, powders and granules. A preferred method of administration consists in using a suitable form containing from 1 mg to about 500 mg of active substance.
The active ingredients can also be administered parenterally in the form of solutions or suspensions for intravenous or intramuscular perfusions or injections. In that case, the compounds according to the invention are generally administered at the rate of about 10 µg to 10 mg per day per kg of body weight; a preferred method of administration consists of using solutions or suspensions containing approximately from 0.01 mg to 1 mg of active substance per ml.

The active ingredients of the invention can be used in a substantially similar manner to other known anti-viral agents, in particular antiretroviral agents. For the polypeptides of this invention, the anti-viral dose to be administered, whether a single dose, multiple dose, or a daily dose, will of course vary with the particular polypeptide employed because of the varying potency of the polypeptide, the chosen route of administration, the size of the recipient, and the nature of the patient's condition. The dosage to be administered is not subject to definite bounds, but it will usually be an effective amount, or the equivalent on a molar basis of the pharmacologically active free form produced from a dosage formulation upon the metabolic release of the active drug to achieve its desired pharmacological and physiological effects. Someone skilled in the art of HIV infection treatment and the like will be able to ascertain, without undue experimentation, appropriate protocols for the effective administration of the polypeptides of this present invention or nucleic acids encoding the same, such as by referring to the earlier published studies on compounds found to have anti-HIV properties or more generally antiviral properties.

Preferably, the pharmaceutical preparation is in a unit dosage form. In such form, the preparation is subdivided into suitable sized unit doses containing appropriate quantities of the active polypeptides, e.g., an effective amount to achieve the desired purpose.

The quantity of the active ingredient in a unit dose of preparation may be generally varied and adjusted from about 0.2 milligrams to about 1000 milligrams, preferably from about 0.5 to about 950 milligrams, more preferably from about 1.0 to about 500 milligrams, and typically from about 1 to 250 milligrams, according to the particular application. The actual dosage employed may be varied depending upon the patient's age, sex, weight and severity of the condition being treated. Such techniques are well known to those skilled in the art.
Generally, the human oral dosage form containing the active ingredients can be administered 1 or 2 times per day. The amount and frequency of the administration will be regulated according to the judgment of the attending clinician. A generally recommended daily dosage for oral administration may range from about 1.0 milligram to about 1,000 milligrams per day, in single or divided doses.

Conventional methods for preparing tablets are known. Such methods include dry methods such as direct compression and compression produced by compaction, or wet methods or other special procedures. Conventional methods for making other forms for administration such as, for example, capsules, suppositories and the like are also well known.

The active ingredients of the invention can be used alone or in combination with other antiviral agents. The other active ingredient can be administered in combination with a compound of the present invention either simultaneously, separately, or sequentially.
By "simultaneous", it is meant that the two compounds are administered at the same time, though not necessarily in the same composition. By "sequential", it is meant that the two compounds are administered within a time period such that the first of the two compounds is still active in the patient when administration of the second of the two compounds occurs.
Preferably, "sequential" means within the same 24 hour, preferably within the same 12 hour, such as within the same 6, 3, 1, half or quarter hour time period.

In an alternate embodiment, this invention provides a method for investigating virus, in particular retrovirus (e.g., HIV), infection and particularly the process of transmission of virus (e.g., HIV)., said methods implementing *in vitro* cultures of human peripheral blood cells (lymphocytes and/or macrophages) and polypeptides described herein.
In this respect, the present invention provides a method for screening and/or characterizing compounds that present anti(retro)viral activity, in particular anti-HIV activity, by implementing *in vitro* cultures of human peripheral blood cells described herein. In particular said methods comprise:
a) contacting a test compound with the *in vitro* cultures of peripheral blood cells in presence of (retro)virus (e.g. HIV) or active part thereof, and
b) determining the anti(retro)viral activity of the test compound in comparison with the anti(retro)viral activity of one of the polypeptides of the present invention.

In another embodiment, the invention discloses methods for inhibiting transmission of virus, in particular retrovirus (e.g. HIV), in a mammal comprising the step of administrating to said mammal any of the pharmaceutical compositions and combinations described above. If the pharmaceutical composition comprises only a polypeptide of this invention as the active component, such methods may additionally comprise the step of administering to said mammal an agent selected from an other anti(retro)viral agent, or an inhibitor of other targets in the (retro)virus life cycle. Such additional agent may be administered to the mammal prior to, concurrently with, or following the administration of the composition of the invention.
Such additional agent may be administered to the mammal prior to, concurrently with, or following the administration of the composition according to this invention.

The polypeptides set forth herein may also be used as laboratory reagents. The polypeptides of this invention may be used to treat or prevent viral contamination of materials and therefore reduce the risk of viral infection of laboratory or medical personnel or patients who come in contact with such materials. These materials include, but are not limited to, biological materials, such as blood, tissue, etc; surgical instruments and garments; laboratory instruments and garments; and blood collection apparatuses and materials.

Further aspects and advantages of this invention will be disclosed in the following examples, which should be regarded as illustrative and not limiting the scope of this application.

### EXAMPLES

### Methods

**Peptide synthesis** - Chemical synthesis of the peptides was performed by the solid phase technique using Fmoc/t-butyl chemistry, as described (Merrifield R.B., Science 232, 341-347, 1986). The crude peptides were purified to homogeneity (> 99%) by C18 reverse-phase high-pressure liquid chromatography. The purified peptides were characterized by amino acid analysis and mass spectrometry (matrix-assisted laser desorption ionisation time-of-flight technique) prior to testing.

**Cell cultures -** Human peripheral blood lymphocytes (PBL) obtained from healthy HIV-seronegative donors were isolated by Ficoll-Hypaque gradient centrifugation and maintained in RPMI 1640 with ultraglutamine supplemented with 20 mg/ml IL2, 100 U/ml penicillin and 100 mg/ml streptamycin, and 10% heat-inactivated fetal calf serum. Cells were stimulated three days in the medium supplemented with 20 U/ml phytohemagglutinin P before evaluation of the antiviral activity.
The human C8166 T-cell line was maintained in RPMI 1640 supplemented with 100 U/ml penicillin and 100 mg/ml streptamycin, and 10% heat-inactivated fetal calf serum.

**Evaluation of antiviral activity on human immune cells -** Samples of 3 x 10⁵ human PBL in 100 ml are preincubated in 96-well plates with various concentrations of each peptide at 37°C (incubator flushed with 5% CO2). After 1 hour incubation, 100 ml of viral suspension (HIV-1 NL4-3 strain) was added at a multiplicity of infection of 1,000 TCID50 per ml. After three washings, cells were cultured in 24-well plates with 1 ml of RPMI 1640 in the continuous presence of the peptides. The cell culture medium was replaced at day 4 post-infection. During this assay, treatment with antiviral compounds was permanent (before, during and after infection). Toxicity was evaluated through daily cell counts and trypan-blue exclusion assays. Infection of human PBL was assessed daily by virus-induced cytopathic effects (syncytium formation) and after 14 days by quantification of p24 antigen in culture supernatants. Measurements of HIV-1 p24 concentrations in the culture supernatant were achieved by ELISA with a detection cut-off of 5 pg/ml (p24 HIV kit, Perkin-Elmer). Similar assays were performed with human C8166 T-cells. Data on PBL are the mean of two separate experiments realized in duplicate.

### Results

Infection assays on human PBL using HIV-1 NL4-3 (1,000 TCID50) in the presence of the products gave the following 50% (IC50) and 100% (IC100) inhibitory concentration values:
Crixivan® (or Indinavir Sulfate):
   (protease inhibitor from Merck Sharp & Dohme)
   IC50 = 0.07 µM
   IC100 = 0.5 µM
Epivir® (or Lamivudine or 3TC):
   (nucleoside reverse transcriptase inhibitor from Glaxo Smithkline Beecham)
   IC50 = 0.05 µM
   IC100 = 0.5 µM
YSPWTNF-NH2 (SEQ ID No. 9):
   (most active peptide)
   IC50 = 0.01 µM
   IC100 = 0.5-1 µM
YSPITNF-NH2 (SEQ ID No. 7):
   IC50 = 0.3 µM
   IC100 = 0.5-1 µM
YdSdPdWdTdNdFd-NH2 :
   (Xd is a D-amino acid residue)
   IC50 = 5 µM
   IC100 = Not determined
YSPWTNF-valerate-NH2 (SEQ ID No. 10):
   IC50 = 0.5 µM
   IC100 = >5 µM
(YSPWTNF)4-K2-K-NH2 :
   IC50 = 1 µM
   IC100 = >5 µM

The peptides are not cytotoxic to human PBL at the maximal peptide concentration tested of 5 µM.
They are not toxic in vivo by intracerebroventricular injection in mice (C57/BL6) up to 200 µg.

## Claims

1. Use of an effective amount of at least one polypeptide comprising at least one amino acid sequence of the general formula (I) : YX₁PX₂TNF (I) (SEQ ID No. 1), wherein X₁ and X₂, independently from each other, are any amino acid residues; any other polypeptide-mimicking molecule; or one nucleic acid encoding the same, for the preparation of pharmaceutical composition intended for the treatment of a disease associated with an infection by a virus.

2. Use according to claim 1, wherein the virus is a retrovirus.

3. Use according to claim 1, wherein the virus is HIV-1 or HIV-2.

4. Use according to claim 1, wherein the virus is HCV, HBC, cytomegalovirus, or an oncogenic virus, in particular human T-cell leukemia viruses (HTLV-I,-II,-III), or feline leukemia virus.

5. Use according to any one of the preceding claims, wherein the polypeptides are from three amino acids to twenty amino acids in length, more preferably from five amino acids to fifteen in length, in particular seven amino acids.

6. Use according to any one of the preceding claims, wherein X₁ is of hydrophilic nature, such as S, K, R, D, E, and P; in particular X₁ is S or K, more particularly S.

7. Use according to any one of the preceding claims, wherein X₂ is of hydrophobic nature, such as C, I, W, V, Y, L, and F ; in particular X₂ is C, I or W.

8. Use according to any one of the preceding claims, wherein the polypeptide presents either carboxylic (e.g., -COOH) or carboxyl-aminated (e.g., -CONH₂) C-terminal extremity.

9. Use according to any one of the preceding claims, wherein the polypeptide presents a lipid moiety moiety, covalently or not covalently attached, at N- or C- terminal extremity.

10. Use according to any one of the preceding claims, wherein the polypeptide comprises at least two amino acid sequences of the general formula (I), said sequences are linear- or branched- attached.

11. Use according to any one of the preceding claims, wherein the polypeptide comprises at least one D-amino acid.

12. Use according to any one of the preceding claims, wherein the polypeptide is selected in the group consisting of YSPITNF (SEQ ID No. 2), YSPITNF-NH₂ (SEQ ID No. 7), YSPCTNF (SEQ ID No. 3), reduced or oxidized YSPCTNF-NH₂ (SEQ ID No. 8), YSPWTNF (SEQ ID No. 4), YSPWTNF-NH₂ (SEQ ID No. 9), YKPITNF (SEQ ID No. 5), YKPITNF-NH₂ (SEQ ID No. 11), YSPWTNF-valerate-NH₂ (SEQ ID No. 10), (YSPWTNF)₄-K₂-K-NH₂, and Y_{D}S_{D}P_{D}W_{D}T_{D}N_{D}F_{D}-NH2.

13. Use according to any one of the preceding claims, wherein the nucleic acid encoding a polypeptide as defined in claim 1 is a DNA molecule comprising the following sequence: TAT TCG CCG TGG ACC AAT TTT (SEQ ID No. 6).

14. An isolated polypeptide selected in the group consisting of YSPITNF, YSPITNF-NH₂, YSPCTNF, reduced or oxidized YSPCTNF-NH₂, YKPITNF, YKPITNF-NH₂, YSPWTNF-NH₂, YSPWTNF-valerate-NH₂, (YSPWTNF)₄-K₂-K-NH₂, and Y_{D}S_{D}P_{D}W_{D}T_{D}N_{D}F_{D}-NH2.

15. An isolated polypeptide of general formula (II):
(YX₁PX₂TNF)₄-K₂-K), wherein X₁ and X₂ are as defined in claim 1 or 7.

16. A polypeptide according to claim 15, which is (YSPWTNF)₄-K₂-K-NH₂.

17. A pharmaceutical composition comprising at least one compound as defined in any of the preceding claims 14-16 and a pharmaceutically acceptable vehicle or support.

18. A pharmaceutical composition according to the preceding claim, said composition further comprising at least one other antiviral agent or inhibitor of other targets in the virus life cycle, or combinations thereof.

19. A pharmaceutical composition according to claim 17 or 18, useful for treating a disease related to an infection by a virus, in particular retrovirus, including, for example, HCV, HBC, cytomegalovirus, and oncogenic viruses, such as human T-cell leukemia viruses (HTLV-I,-II,-III), and feline leukaemia virus, and more particularly HIV-1 or HIV-2.

20. A pharmaceutical composition according to any one of claims 17 to 19, useful for treating HIV infection.

21. A pharmaceutical composition according to any one of claims 17 to 20, useful for treating HIV-1 or HIV-2 infection and complications thereof, in particular AIDS.

22. A method for screening and/or characterizing compounds that present antiviral activity, said method comprises:
c) contacting a test compound with the *in vitro* cultures of peripheral blood cells in presence of virus or active part thereof, and
d) determining the antiviral activity of the test compound in comparison with the antiviral activity of one of the polypeptides as defined in any one the preceding claims 1-16.

23. Use of at least one polypeptide as defined in any of the preceding claims 1-16 as an agent to treat or prevent viral contamination of materials.
